# FASCICULE DE BREVET EUROPEEN

(11) **EP 3 349 699 B1**
(45) Date de publication et mention de la délivrance du brevet: **03.07.2019**
(21) Numéro de dépôt: 16766933.2
(22) Date de dépôt: 14.09.2016
(51) Int. Cl.: A61F 5/56

(54) **DISPOSITIF INTRABUCCAL DESTINÉ AU REPOSITIONNEMENT ET À LA RÉÉDUCATION DE LA LANGUE**
INTRAORALE VORRICHTUNG ZUR REPOSITIONIERUNG UND REHABILITATION DER ZUNGE
INTRAORAL DEVICE FOR THE REPOSITIONING AND REHABILITATION OF THE TONGUE

(30) Priorité: 17.09.2015 FR 1558729
(43) Date de publication de la demande: 25.07.2018
(73) Titulaire: Alglave, Michel, 97440 Saint-André (Ile de la Réunion) (FR)
(72) Inventeur: Alglave, Michel, 97440 Saint-André (Ile de la Réunion) (FR)
(74) Mandataire: Brizio Delaporte, Allison
(86) Numéro de dépôt international: PCT/EP2016/071734
(87) Numéro de publication internationale: WO 2017/046184

(56) Documents cités:
- WO-A1-2014/012827
- US-A1- 2002 144 685
- US-A1- 2008 041 396
- US-A1- 2012 145 166
- US-B1- 6 295 988

## Description

### DOMAINE DE L'INVENTION

L'invention concerne un dispositif intrabuccal destiné au repositionnement et à la rééducation de la langue.

La présente invention trouvera son application dans le domaine orthodontique et plus généralement dans toutes les pathologies en rapport avec une mauvaise position de la langue.

### ETAT DE LA TECHNIQUE

Le positionnement de la langue joue un rôle essentiel dans la croissance de la face et dans les fonctions buccales (mastication, phonation, déglutition, etc.). Une mauvaise position de la langue engendre de nombreux problèmes. Notamment, une position basse de la langue, c'est-à-dire lorsque la pointe de la langue se place en arrière des incisives inférieures, pouvant être combinée ou non à une respiration buccale, est la cause principale des dysmorphoses dento-maxillaires, des malpositions dentaires, des retards et asymétries de croissance de la face, du manque d'oxygénation de la région frontale du cerveau, des troubles du sommeil : ronflements, hypopnée, apnée du sommeil, des dysfonctionnement de l'articulation temporo-mandibulaire (ATM) ou encore des troubles de la prononciation.

Il existe de nombreux dispositifs destinés à inciter la langue à monter dans le palais en arrière de la racine des incisives centrales supérieures.

Ces dispositifs sont classiquement des gouttières orthodontiques en forme de U. Certains comprennent un élément de proprioception, par exemple une languette, se positionnant au niveau du palais pour stimuler la pointe de la langue et l'inciter à rester en haut. Ces dispositifs n'ont qu'un effet temporaire.

D'autres dispositifs comprennent un pan incliné disposé dans la partie mandibulaire permettant de guider la langue en direction du palais en lui procurant un point d'appui. Cependant, les résultats ne sont pas homogènes car la langue a le choix de rester en bas sur le plan incliné, dans une position très confortable, ou de placer la pointe de la langue au niveau du palais ce qui est une position nettement plus fatiguante. Le patient se lasse vite et la langue reste en position basse.

Aucun de ces dispositifs ne maintient la langue dans le palais lors de la déglutition qui intervient environ 2000 fois par 24h.

Le document US 6,295,988 divulgue un dispositif intrabuccal ayant un élément plateau qui permet de maintenir la langue contre le palais du patient.

Il existe donc le besoin de proposer un dispositif qui permet un bon positionnement de la langue tout en assurant la pérennité de la position adéquate.

### RESUME DE L'INVENTION

A cet effet, l'invention concerne un dispositif intrabuccal pour le positionnement de la langue, comprenant un support apte à être fixé en position relativement à l'arcade dentaire maxillaire d'un patient. Le dispositif comprend une lame montée sur le support. La lame s'étendant suivant une direction longitudinale apte à s'inscrire dans un plan sagittal du patient depuis une extrémité postérieure vers une extrémité antérieure. De manière caractéristique, la lame présente une portion élastiquement déformable configurée pour prendre alternativement une position de repos dans laquelle la portion se positionne au-delà d'un plan de morsure et une position contrainte dans laquelle la portion est déformée en direction du plan de morsure.

Avantageusement, la lame comprend un segment s'étendant de l'arrière vers l'avant ayant une composante orientée dans un plan sagittal et dirigée au-delà du plan de morsure.

La lame de l'invention permet de dévier la pointe de la langue vers le haut et l'avant du palais tout en assurant également sa position lors des déglutitions.

Cette lame est destinée à guider la pointe de la langue en position haute, ainsi qu'à la contraindre d'y rester. Si la langue ne se positionne pas correctement en position haute, la portion déformable élastiquement est déformée par la langue, position contrainte de la lame qui repousse alors la langue dans la bonne position en revenant ainsi dans sa position de repos. La pointe de la langue repose avantageusement sur la lame.

Le dispositif permet un positionnement correct de la langue tout en assurant cette position dans la durée.

Le dispositif selon l'invention présente l'avantage d'empêcher toute autre position néfaste de la langue.

Avantageusement, l'extrémité postérieure de la lame est configurée pour s'appliquer sur le plancher buccal et ainsi avantageusement délimiter un compartiment inférieur entre le plancher buccal et la lame qui est non accessible à la langue.

### BREVE DESCRIPTION DES FIGURES

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.
La figure 1 est une vue du dessus du dispositif selon l'invention.
La figure 2 est une vue de la face arrière du dispositif selon l'invention.
La figure 3 est une vue de détail selon la coupe AA de la figure 2.
La figure 4 est une vue du dessous du dispositif selon l'invention.
La figure 5 est une vue de la face avant du dispositif selon l'invention.
La figure 6 est une vue selon la coupe BB de la figure 5.
La figure 7 est une vue du détail C de la figure 6 suivant un mode de réalisation.
Les figures 8a à 8c sont des vues en coupe représentant les mouvements de la lame du dispositif selon l'invention lorsque la langue appuie sur cette lame avant de se positionner en position haute.
Les figures 9a et 9b sont des vues du dessus représentant le positionnement de la langue sur la lame. La figure 9a correspond à la figure 8a et la figure 9b correspond à la figure 8c.

### DESCRIPTION DETAILLEE

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention porte sur un dispositif intrabuccal destiné au repositionnement de la langue, comprenant un support fixable relativement à une arcade dentaire maxillaire d'un patient, le dispositif comprenant une lame montée sur le support et s'étendant suivant une direction longitudinale apte à s'inscrire dans un plan sagittal du patient, depuis une extrémité postérieure vers une extrémité antérieure, caractérisé en ce que la lame comprend une portion déformable élastiquement configurée pour prendre alternativement une position de repos dans laquelle la portion se positionne au moins en partie au-delà d'un plan de morsure du patient et au moins une position contrainte dans laquelle la portion déformée en direction du plan de morsure.

Avantageusement, la portion présente un segment s'étendant de l'arrière vers l'avant ayant une composante orientée dans un plan sagittal et s'éloignant du plan de morsure.

Avantageusement, la portion déformable élastiquement est au moins partiellement courbe.

Avantageusement, la portion déformable élastiquement comprend une concavité opposée au plan de morsure.

Avantageusement, l'extrémité postérieure de la lame est configurée pour s'appliquer sur le plancher buccal.

Avantageusement, l'extrémité postérieure de la lame est configurée pour délimiter un compartiment inférieur entre le plancher buccal et la lame non accessible à la langue.

Avantageusement, le dispositif comprend des moyens de réglage de la portion de la lame.

Avantageusement, les moyens de réglage comprennent des moyens assurant un coulissement de la lame dans des rainures formées dans le support.

Avantageusement, les moyens de réglage de la portion de la lame assurent un réglage de la longueur de la lame (1) de sorte à être atraumatique pour le plancher buccal.

Avantageusement, en position contrainte, la portion déformée se positionne au-delà du plan de morsure.

Avantageusement, en position de repos, la portion déformable élastiquement comprend une surface configurée pour supporter la langue.

Avantageusement, la surface configurée pour supporter la langue est lisse.

Avantageusement, la lame comprend une portion antérieure configurée pour être concave en position de repos et en position contrainte de sorte à assurer le retour en position de repos de la lame.

Avantageusement, l'extrémité postérieure de la lame comprend un bord arrondi présentant une échancrure médiane configurée pour laisser passer le frein de la langue.

Avantageusement, un bord arrondi de la lame comprend deux languettes espacées par l'échancrure médiane et configurées pour s'appliquer sur le plancher buccal.

Avantageusement, la lame comporte une portion distale s'étendant jusqu'à l'extrémité postérieure et ayant une courbure opposée à celle de la portion déformable élastiquement.

Avantageusement, la portion présente une forme cintrée dans un plan perpendiculaire à sa concavité.

Avantageusement, le dispositif comprend au moins un point de fixation avant de la lame au niveau d'une zone antérieure du support.

Avantageusement, le dispositif comprend au moins un point de fixation arrière de la lame au niveau d'une zone postérieure du support.

Avantageusement, entre les points de fixation avant et arrière, la lame est libre de mouvement.

Avantageusement, le dispositif comprend des moyens de fixation de la lame au support au niveau des points de fixation.

Avantageusement, la lame est amovible.

Suivant un aspect séparable, l'invention concerne un dispositif intrabuccal destiné au repositionnement de la langue, comprenant une gouttière dotée d'une face d'appui maxillaire et de deux demi-arcs symétriques de part et d'autre d'une direction médiane et se rejoignant en une zone avant de la gouttière, caractérisé en ce qu' il comprend une lame s'étendant depuis la zone avant vers l'extrémité arrière des deux demi-arcs, la lame faisant au moins partiellement saillie au-dessus de la face d'appui maxillaire et comprenant un segment s'étendant de l'arrière vers l'avant ayant une composante orientée dans un plan sagittal et dirigée au-delà de la face d'appui maxillaire.

Avantageusement, la lame s'étend depuis la zone avant vers l'extrémité arrière des deux demi-arcs et la lame présente une portion élastiquement déformable configurée pour prendre alternativement une position de repos dans laquelle la portion se positionne au moins en partie au-delà de la face d'appui maxillaire et au moins une position contrainte dans laquelle la portion est déformée en direction de la face d'appui maxillaire.

Dans la description qui suit et à moins qu'il en soit indiqué autrement, on entend par symétrique le fait que le dispositif de l'invention présente avantageusement une symétrie, soit pour certaines parties suivant un axe de direction médiane qui sera explicité plus loin, soit suivant un plan du type sagittal. Le terme symétrique ne signifie pas que de légères variations dimensionnelles ou géométriques soient impossibles autour d'une symétrie au sens strict. Notamment, suivant les corrections à apporter à la morphologie de l'utilisateur, le dispositif peut présenter de légères variations entre les parties latérales gauche et droite de l'invention aptes à coopérer avec les arcades dentaires. Le terme « plan sagittal » s'entend de sa définition anatomique habituelle comme d'un plan vertical et perpendiculaire au plan vue de face. Typiquement, ce plan est parallèle à un axe antéropostérieur de la tête et passe par un point situé à midistance entre les deux yeux. Dans le cadre de l'invention, la direction médiane est incluse dans le plan sagittal, le plan sagittal pouvant être défini par ailleurs comme le plan contenant ladite direction médiane et sensiblement perpendiculaire à la face d'appui maxillaire qui sera présentée plus en détails ultérieurement. L'adjectif « frontal » est ici employé pour désigner une direction d'intersection entre un plan frontal et le plan sagittal, c'est-à-dire encore généralement une direction verticale.

Les termes du type sous, en dessous, en deçà s'entendent d'une position relativement à une direction verticale généralement suivant l'épaisseur du support, plus précisément de la gouttière.

On entend par secteur d'arcade dentaire, une portion anatomique de l'utilisateur pouvant comprendre une partie osseuse et/ou une partie dentaire au niveau de laquelle le dispositif peut s'appliquer.

L'adjectif « maxillaire » est utilisé en référence à la mâchoire supérieure et l'adjectif « mandibulaire » en référence à la mâchoire inférieure.

Les termes arrière et postérieur ainsi que avant et antérieur sont utilisés de manière similaire et s'entendent suivant une direction s'inscrivant dans le plan sagittal. L'avant étant situé au niveau des incisives tandis que l'arrière est situé au fond de la cavité buccale.

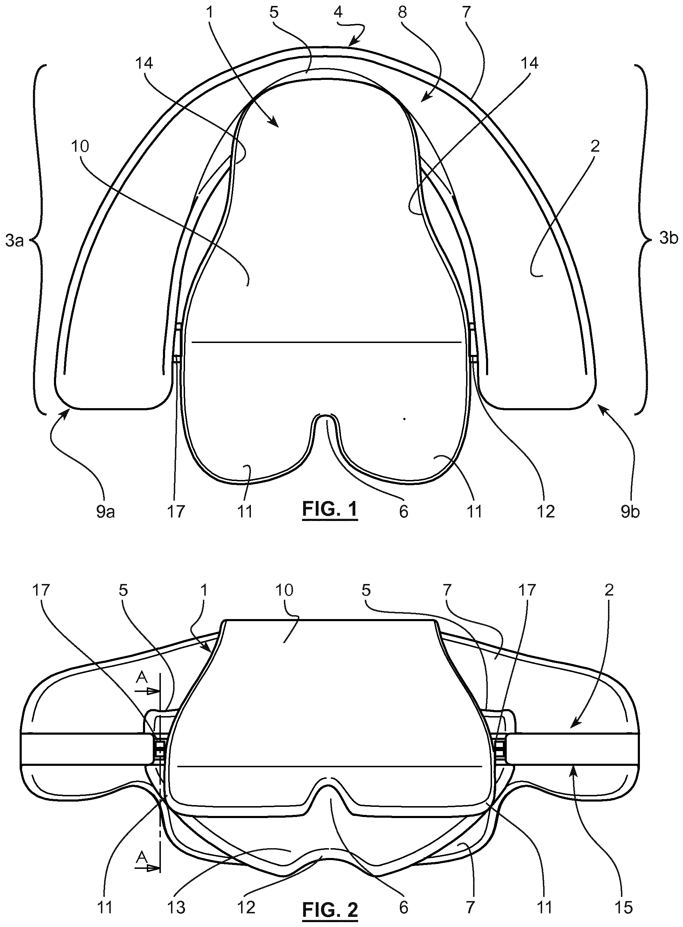

Le plan de morsure est le plan de contact entre l'arcade maxillaire et l'arcade mandibulaire. Dans le mode de réalisation illustré aux figures, le plan de morsure est formé par la gouttière 8 entre la face d'appui maxillaire 2 et la face d'appui mandibulaire 15. Le dispositif selon l'invention apparaît sous différentes vues aux figures 1 à 9. Sur ces figures illustrant un mode de réalisation préféré, le support est une gouttière 8décrite ci-après.

Le support selon l'invention est fixable à l'arcade maxillaire. On entend par fixable que le support est configuré pour être fixe en position au niveau de l'arcade maxillaire. Le support peut être amovible, il n'est pas forcément encastré sur l'arcade maxillaire.

A titre d'exemple, le support est une plaque palatine encore dénommée faux-palais. La plaque palatine est une plaque couramment en résine sur laquelle sont fixés des crochets et ressorts destinés à coopérer avec les dents de l'arcade maxillaire pour maintenir le support en position.

La lame 1 selon l'invention est montée sur la plaque palatine. Différents type de montage peuvent être prévu ; par exemple, la lame 1 est reliée à la plaque palatine par des points de fixation latéraux disposés sensiblement au regard des incisives latérales supérieures ou des canines supérieures et au niveau des premières molaires supérieures. La fixation de la lame 1 sur la plaque palatine peut présenter une élasticité facilitant la déformation de la lame 1 et la mise en place de la langue entre la lame 1 et la plaque palatine. La lame 1 est avantageusement amovible.

Suivant un autre exemple, le support est formé par des bagues dentaires. La lame 1 est montée sur les bagues par des points de fixations latéraux similaires à ce qui est décrit ci-dessus pour la plaque palatine. La lame 1 est avantageusement amovible.

Suivant encore un autre exemple, le support est un arc pouvant être de contention fixé aux dents. La lame 1 est montée sur l'arc ou sur les bagues d'attache de l'arc par des points de fixations latéraux similaires à ce qui est décrit ci-dessus pour la plaque palatine. La lame 1 est avantageusement amovible.

De manière générale, la lame 1 est de préférence montée sur le support de sorte qu'une fois le support en position fixée sur l'arcade maxillaire du patient, les points de fixation de la lame 1 sur le support, au moins à l'avant, se situent avantageusement au niveau des incisives supérieures ou éventuellement des canines et au niveau des premières molaires, à l'arrière de la lame 1, la lame 1 étant libre entre les points de fixation avant et arrière.

Le support présente globalement une forme adaptée aux contours de l'arcade dentaire supérieure de l'utilisateur destiné à recevoir le dispositif.

Comme pour les dispositifs de positionnement de langue connus, le dispositif de l'invention comporte suivant un mode de réalisation une gouttière 8 permettant au moins un appui de la mâchoire supérieure. A cet effet, la gouttière 8 comprend une face d'appui maxillaire 2. Avantageusement mais non exclusivement, la face d'appui maxillaire 2 est sensiblement plane. La face d'appui maxillaire 2 permet l'application des dents de l'arcade dentaire supérieure de l'utilisateur.

A l'opposé de la face d'appui maxillaire 2, la gouttière 8 comporte préférentiellement une face d'appui mandibulaire 15 qui peut être de configuration sensiblement identique à la face d'appui maxillaire 2 c'est à dire préférentiellement plane.

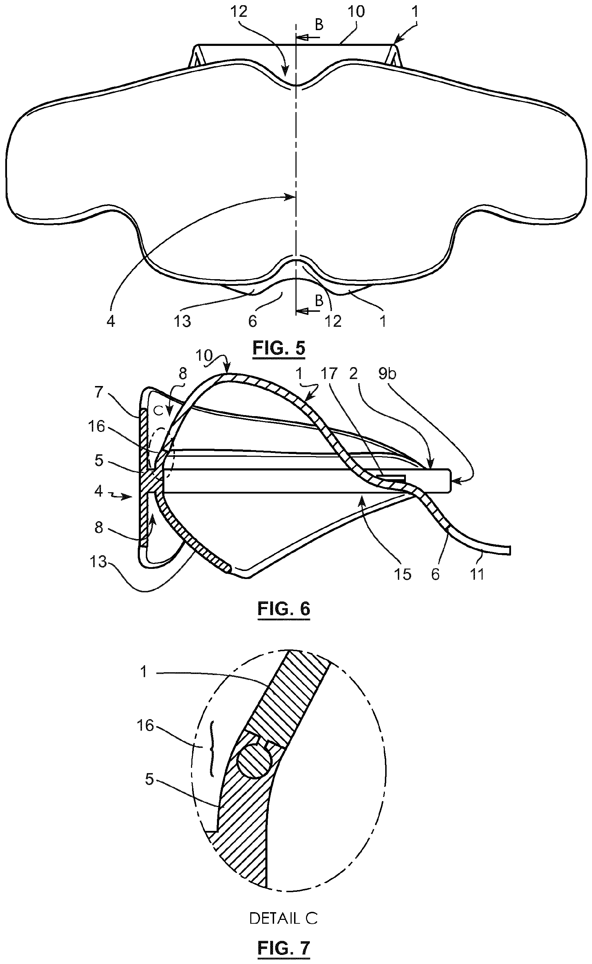

D'une manière générale, pour suivre le contour anatomique de l'arcade dentaire supérieure de l'utilisateur, la gouttière 8 comporte deux demi-arcs 3a, 3b, chacun placé d'un côté de la gouttière 8 autour d'une zone avant 4 réalisant la jonction entre les deux demi-arcs 3a, 3b symétriques. L'axe de symétrie des deux demi-arcs 3a, 3b correspond à une direction médiane m de la gouttière qui la coupe en deux parties égales. Cette direction « m » est visible notamment sur la vue de face, figure 5, et correspond au plan de coupe BB. Cette direction médiane « m » correspond à l'intersection entre le plan sagittal et un plan sensiblement horizontal dans lequel se situe au moins approximativement la face d'appui maxillaire 2.

Les deux demi-arcs 3a, 3b se terminent à l'opposé de la zone avant 4 par des extrémités postérieures 9a, 9b correspondant sensiblement aux dernières molaires du patient.

On comprend aisément que la forme et la dimension de la gouttière 8 sont adaptées à la morphologie de l'utilisateur et peuvent donc être variables. En conséquence, l'invention est ainsi déclinable en plusieurs tailles. La gouttière 1 est avantageusement souple et donc déformable élastiquement comme indiqué précédemment et peut être réalisée dans différents matériaux biocompatibles et particulièrement du silicone.

Dans la partie présentant la face d'appui maxillaire 2 et la face d'appui mandibulaire 15, la gouttière 8 constitue un plan de morsure s'interposant entre l'arcade du haut et celle du bas. Son épaisseur typique est de l'ordre de 2 à 4 millimètres.

La gouttière 8 comprend un écran 7 et avantageusement une bordure postérieure supérieure 5. Cette bordure 5 ainsi que l'écran 7 se positionnent principalement dans la zone avant 4 de la gouttière 8. La bordure postérieure supérieure 5 est la bordure agencée à l'intérieure de la concavité de la gouttière 8 tandis que l'écran 7 est agencé à l'extérieure de la concavité de la gouttière 8. L'écran 7 est par exemple destiné à maintenir les lèvres à distance des dents. Avantageusement la bordure supérieure postérieure 5 et l'écran 7 suivent la convexité de la gouttière 8. La bordure 5 et l'écran 7 s'étendent sensiblement dans un plan perpendiculaire au plan de la face d'appui maxillaire 2. La bordure 5 et l'écran 7 forment des guides antérieur et postérieur pour l'arcade dentaire maxillaire, notamment pour guider les dents dans la gouttière 8.

L'écran 7 est situé dans la zone antérieure du dispositif de l'invention et constitue la partie frontale de l'ensemble. Sa forme est avantageusement convexe. Avantageusement, la convexité est exécutée non seulement suivant des plans parallèles au plan sagittal mais aussi suivant des plans perpendiculaires. L'écran 7 présente une convexité dans le plan sagittal et dans le plan frontal. Cette convexité de l'écran 7, plus précisément ces deux convexités permettent une avancée du sillon gingivo-labial suivant un plan sagittal. Préférentiellement, l'écran 7 comprend un pan supérieur 8 s'étendant au-delà de la face d'appui maxillaire 1 et avantageusement un pan inférieur s'étendant en-deçà de la face d'appui maxillaire 2. L'écran 7 permet de maintenir à distance les lèvres des dents et évitent ainsi aux lèvres d'exercer une pression néfaste sur les dents.

L'écran 7 peut être muni d'ouverture traversante de sorte à faciliter la circulation d'air.

L'écran 7 comporte une incurvation 12 dans le pan supérieur et une incurvation 12 dans le pan inférieur dont la forme est préférentiellement configurée pour respectivement ménager un espace pour le frein labial supérieur et pour le frein labial inférieur et être atraumatique. Les incurvations 12 forment des points bas au niveau du plan sagittal.

L'écran 7 est destiné à être en contact par sa face externe avec les lèvres supérieure et inférieure de l'utilisateur. Préférentiellement, le pan supérieur est destiné à être au contact de la lèvre supérieure ls tandis que le pan inférieur est destiné à être au contact de la lèvre inférieure li.

La forme convexe de l'écran 7 est également destinée à être atraumatique pour l'utilisateur qui doit porter ce dispositif sur des périodes de temps longues, notamment durant la nuit entière. L'écran 7 est configuré pour s'adapter parfaitement à la forme externe de l'arcade dentaire supérieure.

L'écran 7 s'étend avantageusement sur la quasi-totalité de la longueur de la gouttière 8. La hauteur de l'écran 7 est avantageusement décroissante de la zone avant 4 de la gouttière 8 vers l'extrémité des demi-arcs 3a-3b.

Sur la face opposée à la face d'appui maxillaire 1, la face d'appui mandibulaire 15 est avantageusement présente avec au moins un rebord en saillie autour de ladite face d'appui mandibulaire 15. Selon une possibilité, le dispositif comprend un rebord lingual inférieur 13 apte se loger dans le palais inférieur encore dénommé plancher buccal. Le rebord lingual inférieur 13 s'étendant dans la continuité de la bordure postérieure supérieure 5 de la gouttière 8 en deçà de la face d'appui maxillaire 2. Le rebord lingual inférieur 13 est formé d'une nappe sensiblement incurvée dont la convexité est orientée vers la gouttière 8. Le rebord lingual inférieur 13 est avantageusement de la forme d'un secteur de sphère ou d'hémisphère sur une portion angulaire par exemple inférieure à un quart de sphère. De plus, le rebord lingual inférieur 13 forme un guide de positionnement des dents de l'arcade dentaire mandibulaire et notamment des incisives dans la gouttière 8 au niveau de la face d'appui mandibulaire 15.

Le rebord lingual inférieur 13 s'étend selon un mode de réalisation sur un secteur angulaire plus étroit que le secteur angulaire sur lequel s'étend l'écran 7. A titre d'exemple, le rebord lingual inférieur 13 s'étend sur un secteur angulaire de l'ordre de 30° de la gouttière 8.

Le dispositif selon l'invention comprend de manière caractéristique une lame 1. La description ci-après est donnée pour le mode de réalisation illustré aux figures et peut être appliquée sans difficulté à tout support couvert par l'invention et notamment ceux décrits ci-dessus.

La lame 1 s'étend de l'avant vers l'arrière du dispositif et avantageusement entre les deux demi-arcs 3a, 3b. La lame 1 est agencée au moins partiellement au-dessus de la face d'appui maxillaire 2 assurant ainsi une position haute de la langue. Préférentiellement, la lame 1 comprend un segment s'étendant de l'arrière vers l'avant ayant une composante dans un plan sagittal et orientée vers le haut, c'est-à-dire dans une direction s'étendant au-delà de la face d'appui maxillaire 2. Ce segment de lame 1 est donc orienté de l'arrière vers l'avant et du bas vers le haut de sorte à avantageusement guider de manière atraumatique la pointe de la langue vers une position haute, c'est-à-dire la pointe de la langue en arrière des incisives supérieures au contact du palais. Ce segment permet avantageusement d'empêcher la langue d'être en position basse c'est-à-dire au niveau du plancher buccal.

La lame 1 est configurée pour assurer un positionnement correct de la langue, c'est-à-dire la pointe de la langue en arrière des incisives supérieures au contact du palais. La lame 1 s'étend depuis la zone avant 4 de la gouttière préférentiellement de la bordure postérieure supérieure 5 vers l'extrémité des deux demi-arcs 3a-3b de la gouttière 8. Préférentiellement, la lame 1 est fixée à la gouttière 8 par plusieurs points de fixation, plus précisément avantageusement au moins un point de fixation avant au niveau de la zone avant 4 de la bordure postérieure supérieure 5, correspondant à l'arrière des racines des incisives centrales supérieures lorsque le dispositif est en place dans la bouche du patient. La lame 1 est avantageusement également fixée par au moins deux points de fixation arrière au niveau de l'extrémité des deux demi-arcs 3a-3b, plus précisément à l'extrémité arrière de la bordure postérieure supérieure 5. La lame 1 est fixée au plan de morsure de la gouttière 8 au niveau des faces linguales des premières molaires inférieures lorsque le dispositif est en place dans la bouche du patient. Avantageusement, en dehors de ces points de fixation avant et arrière la lame 1 est libre. Cette disposition facilite la déformation de la lame comme décrit ci-après.

La lame 1 comprend au moins une portion 10 déformable élastiquement. La lame 1 présente avantageusement une hauteur maximale comprise entre 0,7 et 1,5 cm. La hauteur de la lame 1 s'entend entre son point le plus éloigné au-dessus du plan de morsure dans un plan sagittal et ledit plan de morsure. La portion 10 de la lame 1 est configurée pour prendre alternativement une position de repos et préférentiellement au moins une position de tension ou contrainte. La lame 1, en position de tension, est configurée pour revenir automatiquement en position de repos. La lame 1 est dite à mémoire de forme, en ce sens que sa déformation est élastique.

Avantageusement, quelle que soit la position de la lame 1, la portion 10 se positionne au moins en partie au-dessus de la face d'appui maxillaire 2.

Préférentiellement, la portion 10 comprend une concavité opposée à la face d'appui maxillaire 2 en position de repos.

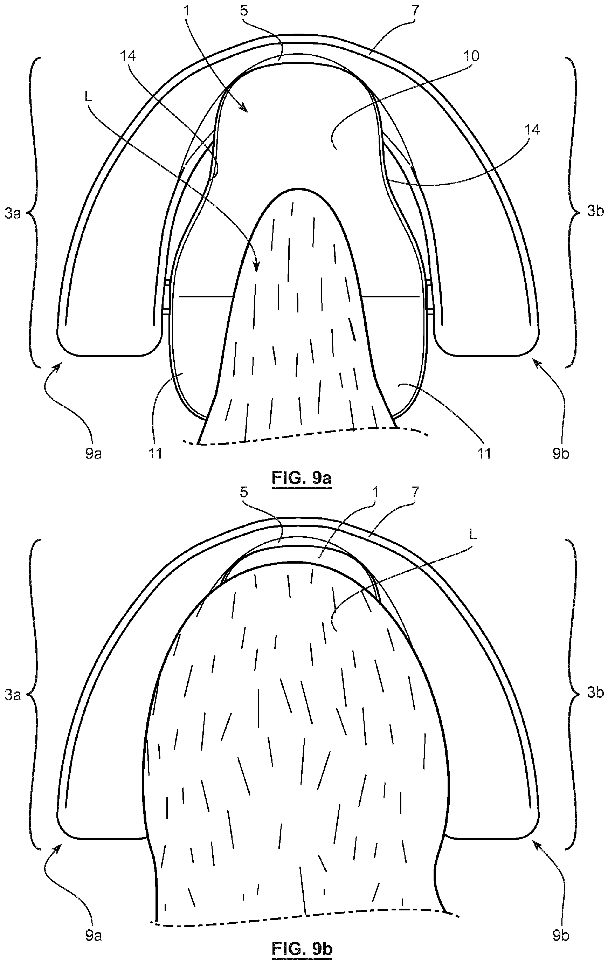

La position de repos est la position stable de la lame 1. Dans la position de repos, la portion 10 concave de la lame 1 est positionnée au moins partiellement au-dessus de la face d'appui maxillaire 2. La concavité est dirigée vers le haut du dispositif c'est-à-dire à l'opposé de la face d'appui maxillaire 2 au-delà de laquelle la portion 10 fait saillie. La portion 10 peut être curviligne, notamment en arc de cercle. Dans cette position de repos, illustrée sur les figures 1 à 7 ainsi que 8c et 9b, la langue est en position haute avec la pointe positionnée au contact du palais supérieur en arrière des incisives. La langue est étalée et présente une pointe arrondie comme illustrée en figure 9b, la langue est aussi plate. La langue est correctement maintenue, même lors des déglutitions.

Avantageusement, la portion 10, préférentiellement au niveau de la concavité, en position de repos de la lame 1 est configurée pour recevoir et supporter la pointe de la langue en position haute. La portion 10 présente avantageusement une surface formant un plateau d'appui pour une zone inférieure de la langue. Préférentiellement, cette surface est lisse de sorte à faciliter la propulsion de la pointe de la langue vers le haut et vers l'avant ainsi qu'à supporter la langue de manière atraumatique.

Avantageusement, cette surface inclut le point le plus haut de ladite portion 10, c'est-à-dire le point le plus éloigné au-dessus du plan de morsure.

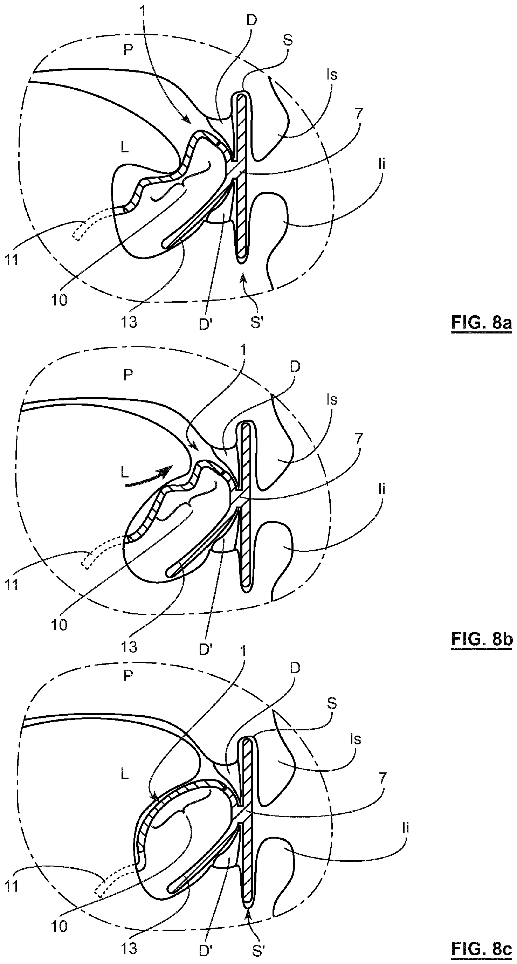

En position de tension, encore dénommée position contrainte, la lame 1 est dans une position déséquilibrée. La portion 10 est déformée. Cette position de lame 1 est illustrée aux figures 8a, 8b et 9a. Selon une possibilité illustrée aux figures 8a et 8b, la portion 10 présente une convexité opposée à la concavité de la portion 10 en position de repos. Avantageusement, la portion 10 déformée est toujours positionnée au-dessus de la face d'appui maxillaire 2.

Préférentiellement, la lame 1 comprend une portion antérieure, c'est-à-dire avantageusement disposée entre le point de fixation avant et la portion 10 concave qui présente également une concavité. Cette concavité est dans le prolongement de la concavité de la portion 10 en positon de repos et reste en forme concave lorsque la portion 10 est déformée en position contrainte. Cette portion antérieure qui reste concave quel que soit la position de la portion déformable élastiquement permet d'assurer cette déformation élastique et la reprise automatique de la position de repos de lame 1.

Avantageusement, la lame 1 forme une cloison séparant la cavité buccale en deux compartiments : un compartiment supérieur, proche du palais et un compartiment inférieur, proche du plancher buccal et bloquant l'accès au compartiment inférieur.

Selon un mode de réalisation particulièrement préféré, la lame 1 comporte une portion distale d'étendant jusqu'à l'extrémité postérieure. La portion distale présente avantageusement, au moins en position de repos, une courbure, ou encore une concavité, opposée à celle de la portion 10. La lame 1 présente préférentiellement, au moins en position de repos, deux courbures opposées. La lame 1 est configurée pour se déformer en accordéon lors d'une poussée de la portion 10 par la langue. Les deux courbures sont soit disposées dans le prolongement l'une de l'autre donnant une forme sensiblement en S de la lame 1, soit la lame comprend une inflexion en direction au niveau du plan de morsure, entre la concavité de la portion 10 et la concavité opposée de l'extrémité arrière de la lame 1.

Suivant un exemple illustré sur les figures, la lame 1 se prolonge au-delà des points de fixation arrières situés à l'arrière du dispositif. Préférentiellement, le prolongement de la lame permet d'assurer un contact avec le plancher buccal. La lame 1 présente avantageusement un bord arrondi et une échancrure 6 médiane configurée pour ménager un espace pour le frein lingual et être atraumatique et formant avantageusement deux languettes 11. La lame 1 se prolonge au-delà des points de fixation, situés à l'arrière du dispositif, et en-dessous de la face d'appui maxillaire 2. Les languettes 11 sont situées sous la face d'appui maxillaire 2. Les languettes 11, c'est-à-dire le bord inférieur atraumatique de la lame 1, sont destinées à être en contact avec le plancher buccal de sorte à empêcher le passage de la langue sous la lame 1. Préférentiellement, la position basse de la langue n'est plus possible car le compartiment inférieur de la cavité buccale n'est plus accessible.

De la zone avant 4 vers l'arrière du dispositif la lame 1 comprend avantageusement la portion antérieure, puis la portion 10 déformable élastiquement, puis le segment orienté de l'arrière vers l'avant et de bas en haut pouvant être éventuellement déformable élastiquement et faire partie de la portion 10, puis la portion distale ou postérieure comprenant suivant une possibilité les languettes 11 et avantageusement l'échancrure médiane 6 pour laisser passer le frein de la langue.

La lame 1 est une lame souple. De préférence, l'épaisseur de la lame 1 est inférieure à 2mm, préférentiellement à 1mm.

L'épaisseur de la lame 1 peut être variée le long d'un axe longitudinal de la lame 1. Par exemple, la lame 1 peut être plus fine à l'extrémité arrière et/ou plus épaisse au niveau de la portion 10 de sorte à être atraumatique et contribuer à la déformation élastique de la portion 10. La lame 1 peut être réalisée dans différents matériaux biocompatibles particulièrement du silicone.

Suivant une possibilité, la lame 1 a une forme cintrée, préférentiellement dans un plan perpendiculaire à la concavité, et avantageusement au niveau de la portion courbe 10. De cette manière, la lame 1 présente une largeur moindre au sommet de la portion 10, en position de repos. La portion 10 présente avantageusement un resserrement 14 de part et d'autre. Cette forme cintrée facilite la déformation de la lame 1 et son retour en position de repos.

De manière générale, la lame 1 est mobile de bas en haut dans la gouttière et présente avantageusement une largeur inférieure à l'espace délimité entre les deux demi-arcs 3a, 3b.

Suivant un mode de réalisation illustré aux figures 6 et 7, la lame 1 est au moins en partie amovible. Le dispositif comprend des moyens de fixation 16 de la lame 1 sur la gouttière 8 et préférentiellement sur la bordure postérieure supérieure 5. Ces moyens de fixation 16 permettent de fixer la lame 1 et donc de former un tremplin à langue telle que décrite ci-dessus pour faciliter le positionnement linguale sur une gouttière 8 de base par exemple sur un appareil d'orthodontie classique.

A titre d'exemple non limitatif, ces moyens de fixation 16 sont par exemple des systèmes males-femelles tels que des encoches et des saillies complémentaires sur la bordure postérieure supérieure 5 tel qu'illustré en figure 7 et sur la lame 1 ainsi qu'aux extrémités 9a, 9b des deux demi-arcs 3a-3b et sur la lame 1. On peut ainsi former une partie de base de dispositif apte à coopérer sélectivement avec une parmi plusieurs lames 8 en particulier pour une adaptation simple à différents anatomies d'utilisateurs. La fixation est rapide et simple.

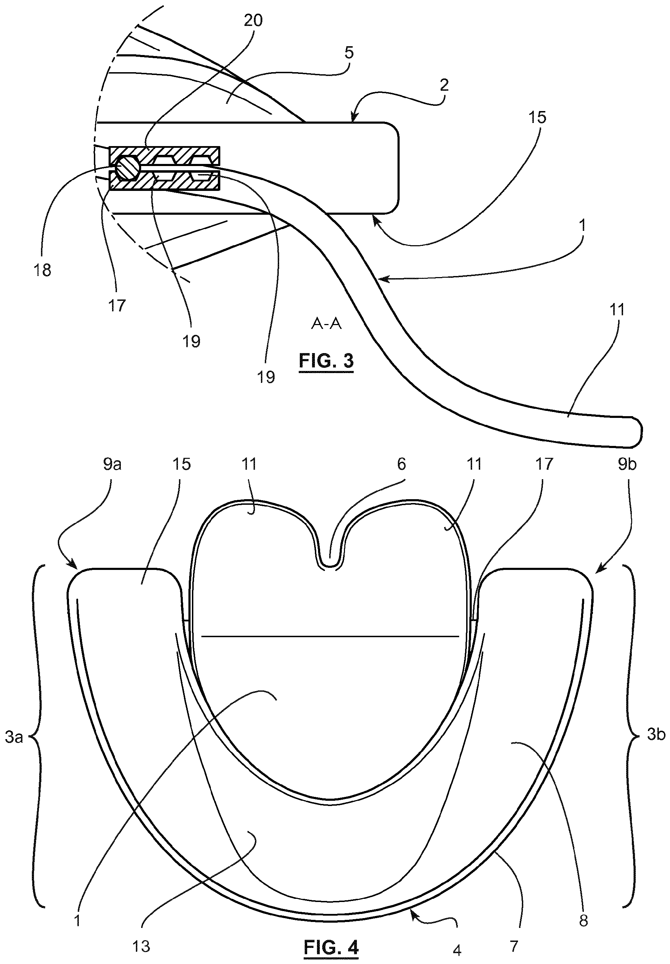

Suivant un mode de réalisation illustré en figure 2 et 3, le dispositif comprend des moyens de réglage 17 de la portion courbe 10. Ces moyens de réglages 17 sont destinés à permettre une modification de la longueur et/ou de la hauteur et/ou de la concavité de la portion courbe 10 de sorte à s'adapter au mieux à la morphologie de chaque patient. A titre d'exemple non limitatif, les moyens de réglage 17 permettent un coulissement de la lame 1 dans des rainures complémentaires formées dans la gouttière 8 préférentiellement sur la face linguale au niveau des racines des premières molaires inférieures. Suivant un autre exemple illustré en figure 3, les moyens de réglage 17 comprennent des moyens de fixation males/femelles et une glissière, telle que par exemple un tenon 18 est formé sur la lame 1 coopérant avec des mortaises 19 dans lesquelles vient se fixer le tenon 18. Les mortaises 19 formées dans la gouttière 8 sont reliées par une glissière 20 dans laquelle glisse le tenon 18.

Après réglage de la longueur de la lame 1, le praticien peut couper l'extrémité inférieure de la lame 1, préférentiellement au niveau des languettes 11, de sorte à limiter tout frottement traumatisant pour le plancher buccal.

On décrit ci-après un mode opératoire de l'invention.

La mandibule de l'utilisateur qui serre les dents se place dans le canal formé par la gouttière 8 au niveau de la face d'appui maxillaire 2. Avantageusement, la gouttière 8 rentre dans la bouche de l'utilisateur en étant contractée transversalement entre le pouce et l'index.

Au préalable, on aura pu régler les dimensions du dispositif ou trouver la taille adéquate à la morphologie de l'utilisateur et en particulier thermoformer la gouttière 8 selon la dimension transversale souhaitée en fonction de la taille du palais de l'utilisateur et en adaptant les dimensions de la lame 1.

La langue se met alors soit directement en position haute en contact de la portion 10 de la lame 1 en position de repos, soit la langue pousse avec sa pointe vers le bas sur la portion 10 de la lame 1 entrainant sa déformation en position de tension. La lame renvoie alors la pointe de la langue vers le haut et vers l'avant pour retrouver sa position de repos idéale et positionner la langue en position haute. Lorsque la langue pousse sur la lame 1 comme illustrée en figure 8a, celle-ci présente une forme étroite et pointue comme illustrée en figure 9a.

La poussée transversale de la langue et son positionnement correcte en position haute permet de rétablir une croissance normale de la face.

Le dispositif selon l'invention est indiqué pour rééduquer la position de la langue au repos, lors de la déglutition, de jour comme de nuit. La nuit, la langue est plaquée au palais et n'aura pas tendance à se relâcher obstruant le pharynx et faisant vibrer la luette générant des ronflements.

### REFERENCES

1. Lame
2. Face d'appui maxillaire
3. a - 3.b demi-arc
4. Zone avant de la gouttière
5. Bordure postérieure supérieure
6. Echancrure médiane
7. Ecran
8. Gouttière
9. a- 9b extrémités postérieures
10. Portion
11. Languette
12. Incurvation
13. Rebord lingual inférieur
14. Resserrement
15. Face d'appui mandibulaire
16. Moyens de fixation
17. Moyens de réglages de la portion courbe
18. Tenon
19. Mortaise
20. Glissière
M. Direction médiane
Ls. Lèvre supérieure
Li. Lèvre inférieure
L. Langue
S. Sillon gingivo labial supérieur
S'. Sillon gingivo labial inférieur
P. Palais
D. Dent - incisive centrale supérieure
D'. Dent - incisive centrale inférieure

## Revendications

1. Dispositif intrabuccal destiné au repositionnement de la langue, comprenant un support fixable relativement à une arcade dentaire maxillaire d'un patient, le dispositif comprenant une lame (1) montée sur le support et s'étendant suivant une direction longitudinale apte à s'inscrire dans un plan sagittal du patient, depuis une extrémité postérieure vers une extrémité antérieure, **caractérisé en ce que** la lame (1) comprend une portion (10) déformable élastiquement configurée pour prendre alternativement une position de repos dans laquelle la portion (10) se positionne au moins en partie au-delà d'un plan de morsure du patient et au moins une position contrainte dans laquelle la portion (10) est déformée en direction du plan de morsure.

2. Dispositif selon la revendication précédente dans lequel la portion (10) présente un segment s'étendant de l'arrière vers l'avant ayant une composante orientée dans un plan sagittal et s'éloignant du plan de morsure (2).

3. Dispositif selon l'une quelconque des revendications précédentes dans lequel la portion (10) déformable élastiquement est au moins partiellement courbe.

4. Dispositif selon l'une quelconque des revendications précédentes dans lequel la portion (10) déformable élastiquement comprend une concavité opposée au plan de morsure (2).

5. Dispositif selon l'une quelconque des revendications précédentes dans lequel l'extrémité postérieure de la lame (1) est configurée pour s'appliquer sur le plancher buccal.

6. Dispositif selon l'une quelconque des revendications précédentes dans lequel l'extrémité postérieure de la lame (1) est configurée pour délimiter un compartiment inférieur entre le plancher buccal et la lame (1) non accessible à la langue.

7. Dispositif selon l'une quelconque des revendications précédentes comprenant des moyens de réglage (17) de la portion (10) de la lame (1).

8. Dispositif selon la revendication précédente dans lequel les moyens de réglage (17) comprennent des moyens assurant un coulissement de la lame (1) dans des rainures formées dans le support.

9. Dispositif selon l'une quelconque des deux revendications précédentes dans lequel les moyens de réglage (17) de la portion (10) de la lame (1) assurent un réglage de la longueur de la lame (1) de sorte à être atraumatique pour le plancher buccal.

10. Dispositif selon l'une quelconque des revendications précédentes dans lequel en position contrainte, la portion (10) déformée se positionne au-delà du plan de morsure (2).

11. Dispositif selon l'une quelconque des revendications précédentes dans lequel en position de repos, la portion (10) déformable élastiquement comprend une surface configurée pour supporter la langue.

12. Dispositif selon l'une quelconque des revendications précédentes dans lequel la lame (1) comprend une portion antérieure configurée pour être concave en position de repos et en position contrainte de sorte à assurer le retour en position de repos de la lame.

13. Dispositif selon l'une quelconque des revendications précédentes en combinaison de la revendication 4 dans lequel la lame (1) comporte une portion distale s'étendant jusqu'à l'extrémité postérieure et ayant une courbure opposée à celle de la portion déformable élastiquement (10).

14. Dispositif selon l'une quelconque des revendications précédentes comprenant au moins un point de fixation avant de la lame (1) au niveau d'une zone antérieure du support.

15. Dispositif selon la revendication précédente, dans lequel entre les points de fixation avant et arrière, la lame (1) est libre de mouvement.

## Patentansprüche

1. Intraorale Vorrichtung zur Repositionierung und Rehabilitation der Zunge, umfassend einen Träger, der mit Bezug auf einen Kieferzahnbogen eines Patienten fixierbar ist, wobei die Vorrichtung eine Klinge (1) umfasst, die auf dem Träger montiert ist und sich gemäß einer Längsrichtung, die ausgelegt ist, um sich in eine saggitale Ebene des Patienten einzuschreiben, von einem hinteren Ende hin zu einem vorderen Ende erstreckt, **dadurch gekennzeichnet, dass** die Klinge (1) einen elastisch verformbaren Abschnitt (10) umfasst, der konfiguriert ist, um alternativ eine Ruheposition, in der sich der Abschnitt (10) mindestens teilweise außerhalb einer Bissebene des Patienten positioniert, und mindestens eine gespannte Position einzunehmen, in der der Abschnitt (10) in Richtung der Bissebene verformt ist.

2. Vorrichtung nach dem vorhergehenden Anspruch, wobei der Abschnitt (10) ein Segment darstellt, das sich von hinten nach vorne erstreckt und einen Bestandteil aufweist, der auf eine saggitale Ebene ausgerichtet ist und sich von der Bissebene (2) entfernt.

3. Vorrichtung nach einem der vorhergehenden Ansprüche, wobei der elastisch verformbare Abschnitt (10) mindestens teilweise gekrümmt ist.

4. Vorrichtung nach einem der vorhergehenden Ansprüche, wobei der elastisch verformbare Abschnitt (10) eine Konkavität gegenüber der Bissebene (2) umfasst.

5. Vorrichtung nach einem der vorhergehenden Ansprüche, wobei das hintere Ende der Klinge (1) konfiguriert ist, um auf den Mundboden angewendet zu werden.

6. Vorrichtung nach einem der vorhergehenden Ansprüche, wobei das hintere Ende der Klinge (1) konfiguriert ist, um ein inneres Abteil zwischen dem Mundboden und der Klinge (1), der für die Zunge nicht zugänglich ist, zu begrenzen.

7. Vorrichtung nach einem der vorhergehenden Ansprüche, umfassend Mittel zum Einstellen (17) des Abschnitts (10) der Klinge (1).

8. Vorrichtung nach dem vorhergehenden Anspruch, wobei die Mittel zum Einstellen (17) Mittel umfassen, die das Gleiten der Klinge (1) in den Nuten, gebildet im Träger, sicherstellen.

9. Vorrichtung nach einem der zwei vorhergehenden Ansprüche, wobei die Mittel zum Einstellen (17) des Abschnitts (10) der Klinge (1) eine Einstellung der Länge der Klinge (1) sicherstellen, um für den Mundboden atraumatisch zu sein.

10. Vorrichtung nach einem der vorhergehenden Ansprüche, wobei in der gespannten Position der verformte Abschnitt (10) außerhalb der Bissebene (2) positioniert ist.

11. Vorrichtung nach einem der vorhergehenden Ansprüche, wobei in der Ruheposition, der elastisch verformbare Abschnitt (10) eine Fläche umfasst, die konfiguriert ist, um die Zunge zu tragen.

12. Vorrichtung nach einem der vorhergehenden Ansprüche, wobei die Klinge (1) einen vorderen Abschnitt umfasst, der konfiguriert ist, um in der Ruheposition und in der gespannten Position konkav zu sein, um die Rückkehr der Klinge in die Ruheposition sicherzustellen.

13. Vorrichtung nach einem der vorhergehenden Ansprüche in Kombination mit Anspruch 4, wobei die Klinge (1) einen distalen Abschnitt umfasst, der sich bis zum hinteren Ende erstreckt und eine Krümmung gegenüber derjenigen des elastisch verformbaren Abschnitts (10) aufweist.

14. Vorrichtung nach einem der vorhergehenden Ansprüche, umfassend mindestens einen Fixierungspunkt vor der Klinge (1) auf der Ebene der vorderen Zone des Trägers.

15. Vorrichtung nach dem vorhergehenden Anspruch, wobei zwischen dem vorderen und hinteren Fixierungspunkt die Klinge (1) frei beweglich ist.

## Claims

1. Intraoral device for the repositioning of the tongue, comprising a fixable support member in relation to a patient's maxillary dental arch, the device comprising a strip (1) mounted on the support member and extending along a longitudinal direction suitable for being inscribed in a sagittal plane of the patient, from a posterior end to an anterior end, **characterised in that** the strip (1) comprises an elastically deformable portion (10) configured to adopt alternately a rest position wherein the portion (10) is positioned at least in part beyond a bite plane of the patient and at least one constrained position wherein the portion (10) is deformed towards the bite plane.

2. Device according to the preceding claim wherein the portion (10) has a segment extending from the rear to the front having a component oriented in a sagittal plane and moving away from the bite plane (2).

3. Device according to any one of the preceding claims wherein the elastically deformable portion (10) is at least partially curved.

4. Device according to any one of the preceding claims wherein the elastically deformable portion (10) comprises a concavity opposite the bite plane (2).

5. Device according to any one of the preceding claims wherein the posterior end of the strip (1) is configured to be applied to the floor of the mouth.

6. Device according to any one of the preceding claims wherein the posterior end of the strip (1) is configured to delimit a lower compartment between the floor of the mouth and the strip (1) not accessible to the tongue.

7. Device according to any one of the preceding claims comprising adjustment means (17) of the portion (10) of the strip (1).

8. Device according to the preceding claim wherein the adjustment means (17) comprise means for sliding the strip (1) in grooves formed in the support member.

9. Device according to any one of the two preceding claims wherein the adjustment means (17) of the portion (10) of the strip (1) adjust the length of the strip (1) so as to be atraumatic for the floor of the mouth.

10. Device according to any one of the preceding claims wherein, in the constrained position, the deformed portion (10) is positioned beyond the bite plane (2).

11. Device according to any one of the preceding claims wherein, in the rest position, the elastically deformable portion (10) comprises a surface configured to support the tongue.

12. Device according to any one of the preceding claims wherein the strip (1) comprises an anterior portion configured to be concave in the rest position and in the constrained position so as to ensure the return of the strip to the rest position.

13. Device according to any one of the preceding claims in combination with claim 4 wherein the strip (1) includes a distal portion extending to the posterior end and having an opposite curvature to that of the elastically deformable portion (10).

14. Device according to any one of the preceding claims comprising at least one front fixing point of the strip (1) at the level of an anterior zone of the support member.

15. Device according to the preceding claim, wherein between the front and rear fixing points, the strip (1) is free to move.
